# EUROPÄISCHE PATENTSCHRIFT

(11) **EP 0 796 712 B1**
(45) Veröffentlichungstag und Bekanntmachung des Hinweises auf die Patenterteilung: **31.07.2002**
(21) Anmeldenummer: 97101670.4
(22) Anmeldetag: 04.02.1997
(51) Int. Cl.: B29C 45/14, A61M 31/00

(54) **Vorrichtung zur Herstellung von perl-schnurförmigen pharmakahaltigen Implantaten**
Apparatus for producing pearls-rope like implants containing medicaments
Dispositif de fabrication d'implants renfermants des médicaments en forme d'un collier de perles

(30) Priorität: 22.02.1996 DE 19606490
(43) Veröffentlichungstag der Anmeldung: 24.09.1997
(73) Patentinhaber: MERCK PATENT GmbH, 64293 Darmstadt (DE)
(72) Erfinder: Klemm, Klaus, Dr., 60435 Frankfurt/Main (DE); Gilberger, Ben, 35492 Giessen (DE)

(56) Entgegenhaltungen:
- EP-A- 0 157 910
- EP-A- 0 225 846
- DE-C- 705 243
- GB-A- 630 364
- GB-A- 2 192 362

## Beschreibung

Die Erfindung betrifft eine Vorrichtung zur Herstellung von perlschnurförmigen pharmakahaltigen Implantaten, bestehend aus Ketten von jeweils auf einem chirurgischen Draht oder Faden aufgereihten pharmakahaltigen Formkörperchen.

Bei der Versorgung von infektionsgefährdeten Operations- urid Verletzungswunden werden häufig Kunststoffimplantate eingesetzt, die antibiotisch wirkende Pharmazeutika enthalten und diese über einen bestimmten Zeitraum hinweg kontinuierlich abgeben. Bei bestimmten Indikationen haben sich pharmakahaltige Kunststoffkörperchen bewährt, die perlschnurartig auf chirurgischen Drähten oder Fäden aufgezogen sind (DE-A-23 20 373, DE-A-26 51 441, EP-A-10 157 909, EP-A-0 157 910).

Ein Vorteil dieser Ketten pharmakahaltiger Körperchen im Vergleich zu anderen Implantaten zur lokalen Wirkstoffabgabe ist, daß der Chirurg durch einfaches Ablängen der Ketten eine an Größe des Wundortes und Wirkstoffbedarf orientierte Dosierung vornehmen kann. Ein weiterer Vorteil ist, daß nach Therapieende oder Erschöpfung die Ketten mit vergleichsweise geringem operativem Aufwand zusammenhängend aus dem Körperbereich entnommen werden können. Als Kunststoffmatrix für die pharmakahaltigen Körperchen dienen in aller Regel Polymermaterialien auf Basis von Poly(meth)acrylaten.

Die industrielle Herstellung derartigen Ketten pharmakahaltiger Kunststoffkörperchen erfolgt in der Weise, daß ein Gemisch aus pulverigem oder granuliertem Poly(meth)acrylat-Kunststoff und dem pharmazeutischen Wirkstoff sowie gegebenenfalls weiteren Zuschlagstoffen extrudiert und im Spritzgußverfahren in einer Form zu beispielsweise kugelförmigen Körperchen ausgeformt werden, wobei sie taktweise auf einen durch die Form geführten Faden oder Draht aufgespritzt werden.

Industriell hergestellte perlschnurförmige Implantate können aus fertigungstechnischen, wirtschaftlichen und nicht zuletzt zulassungsrechtlichen Gründen nicht mit beliebigen pharmazeutischen Wirkstoffen und mit beliebigen Dosierung angeboten werden. Über die Fertigprodukte mit vorgegebener Wirkstoffbeladung hinaus besteht bei den Chirurgen jedoch häufig ein Bedarf, mit Implantaten dieser Art zu therapieren, die eine individuelle Auswahl, Kombination oder Dosierung an Wirkstoff zulassen. Dies kann durch eine Eigenherstellung vor oder während der Operation, bei der die Wirkstoffbeladung nach den fallspezifischen Vorgaben rezeptiert wird, bewerkstelligt werden.

Bekannt ist die Verwendung von auf Poly(meth)acrylat-Kunststoff basierendem Knochenzement als Matrixmaterial für pharmakahaltige Implantate. Knochenzemente sind selbsthärtende Gemische, die in aller Regel im wesentlichen aus einer pulverigen Feststoffkomponente aus Poly(meth)acrylat und einer flüssigen Komponente aus einem (Meth)-acrylat bestehen. Durch Mischen dieser Komponenten können niedrig- bis hochviskose Massen eingestellt werden, die durch die Anwesenheit von Polymerisationsinitiatoren innerhalb eines Zeitraums von typisch ca. 10-20 Minuten aushärten. Derartige Gemische versetzt mit pharmazeutischen Wirkstoffen können ebenfalls zur Herstellung von pharmakahaltigen Implantaten dienen, wobei die noch nicht ausgehärtete Masse in entsprechende Formen gespritzt wird. Diese Herstellungsvariante ist für die manuelle Herstellung geeignet, insbesondere weil die Verarbeitungszeit des Knochenzementgemisches nur kurz ist.

Aufgabe der Erfindung ist es, eine Vorrichtung zur Herstellung von perlschnurförmigen pharmakahaltigen Implantaten zu schaffen, die es ermöglicht, diese Implantate mit individueller Wirkstoffbeladung auch vor oder während einer Operation manuell herzustellen.

Diese Aufgabe wird erfindungsgemäß dadurch gelöst, daß
(i) zwei längliche Formhälften in einem Formgehäuse aufgenommen, mittels einer Positionierungseinrichtung gegeneinander ausgerichtet und durch eine Anpreßeinrichtung mit ihren Formtrennflächen gegeneinander bis auf Dichtigkeit preßbar sind, daß
(ii) in den einander zugekehrten Formtrennflächen mindestens in einer Reihe angeordnete Formnester zur Aufnahme jeweils eines Formkörperchens und eines diese verbindenden Drahtes oder Fadens ausgespart sind, daß
(iii) die Formnester jeweils mit einer an einer Formaußenfläche mündenden Angußöffnung verbunden sind, und daß
(iv) jeweils mehrere Formnester einer Reihe an eine gemeinsame, in einer Innenwand des Formgehäuses ausgesparte Verteilernut angeschlossen sind, in die jeweils eine mit einer Einspritzvorrichtung dicht verbindbare Einspritzöffnung mündet.

Der einfache Aufbau der Vorrichtung ermöglicht die Herstellung der Implantate, wobei der als Matrixwerkstoff verwendete Knochenzement mit dem pharmazeutischen Wirkstoff der jeweils erforderlichen Art, Menge und Zusammensetzung erst unmittelbar vor der Herstellung vermischt wird. Da die beiden Formhälften von dem einfach aufgebauten Formgehäuse umschlossen und gehalten werden, ist die gesamte Vorrichtung von einfachem Aufbau und kann daher in einfacher Weise gehandhabt werden. Der gesamte Arbeitsgang kann von einer Person ausgeführt werden, ohne daß hierfür irgendwelche Spezialwerkzeuge oder zusätzliche Vorrichtungen erforderlich wären.

Wegen ihren einfachen Aufbaus ist die Vorrichtung leicht zu reinigen, so daß eine beliebig häufige Benutzung auch in kurzen Zeitabständen ermöglicht wird. Da jede Einspritzöffnung mit mehreren Formnestern verbunden ist, können mit einem einzelnen Einspritzvorgang mehrere der auf den Draht oder Faden aufgereihten Formkörperchen hergestellt werden.

Die Angußöffnungen sind dabei so plaziert, daß ein möglichst geringer Abstand zwischen Formkörper und Formtrennflächen vorliegt.

Gemäß einer bevorzugten Ausführungsform der Erfindung ist vorgesehen, daß in den beiden Formhälften zwei parallele Reihen von Formnestern ausgespart sind, deren Angußöffnungen in gegenüberliegenden Formaußenfiächen münden, und daß die Verteilernuten und die Einspritzöffnungen in einander gegenüberliegenden Wänden des Formgehäuses angeordnet sind. Damit wird erreicht, daß von zwei Seiten her das Einspritzen vorgenommen und auf diese Weise in einem einzigen Spritzgießvorgang zwei Ketten von Implantaten hergestellt werden können.

Als erfindungsgemäße Anpreßeinrichtung, sind alle Vorrichtungen zu verstehen, die dazu dienen, die Formhälften so fest aneinander zu pressen, daß das in die Form unter Druck über die Einspritzöffnungen eingebrachte fließfähige Material nicht anderswo austreten kann. Hierfür eignen sich in erster Linie Klammern und insbesondere Verschraubungen. Die Anpreßvorrichtung kann sowohl das Formgehäuse mit einbeziehen, als auch nur, wie bevorzugt, die Formhälftenteile alleine umfassen. Bevorzugt sind versenkte Verschraubungen der Formhältenteile senkrecht zu ihren Formtrennflächen (3).

Als erfindungsgemäße Positionierungseinrichtung sind alle Vorrichtungen zu verstehen, die dazu dienen, die Formhälften exakt und rasch zu ihrer Position zueinander zu fixieren, so daß eine optimale Gußform entsteht. Stifte, Bolzen, Noppen, Nute usw. sind hierfür geeignet, die auf der gegenüberliegenden Formhälfte ein entsprechender Pendant in Form einer Vertiefung besitzen müssen. Bolzen und Bolzenlöcher sind zur Positionierung bevorzugt geeignet.

Weitere vorteilhafte Ausgestaltungen des Erfindungsgedankens sind Gegenstand weiterer Unteransprüche.

Nachfolgend wird ein Ausführungsbeispiel der Erfindung näher beschrieben, das in der Zeichnung dargestellt ist. Es zeigt:
Fig. 1 einen Querschnitt durch eine Vorrichtung zur Herstellung von perlschnurförmigen pharmakahaltigen implantaten.
Fig. 2 einen Schnitt längs der Linie II-II in Fig. 1 und
Fig. 3 einen vergrößerten Teilschnitt durch die beiden Formhälften im Bereich ihrer Formtrennflächen.
Fig. 4 einen vergrößerten Teilschnitt (X) durch die beiden Formhälften im Bereich der schraubenartigen Anpreßvorrichtung.

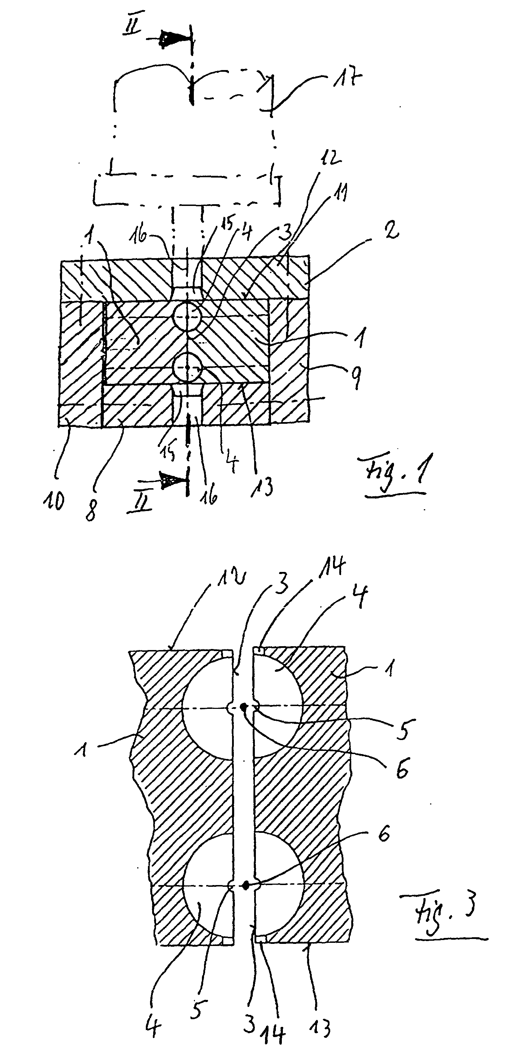

Die in der Zeichnung dargestellte Vorrichtung dient dazu, aus einem Gemisch von Knochenzement und pharmakologischen Wirkstoffen ein perlschnurförmiges pharmakahaltiges Implantat herzustellen, das aus einer Kette von Kunststoffkörperchen besteht, die auf einen chirurgischen Draht oder Faden aufgereiht sind. Wie man aus Fig. 1 erkennt, umschließen zwei längliche Formhälften 1, die in einem Formgehäuse 2 aufgenommen sind, in ihren einander zugekehrten Formtrennflächen 3 zwei Reihen von Formnestern 4, die beim dargestellten Ausführungsbeispiel kugelförmig gestaltet sind. Jede Formnesthälfte in jeder Formhälfte 1 ist deshalb halbkugelförmig. Benachbarte Formnester 4 jeder Reihe sind durch kurze Kanäle 5 verbunden, in die vor dem Schließen der Form ein chirurgischer Faden 6 oder Draht als Trägerseele für das herzustellende Implantat eingelegt wird.

Die beiden Formhälften 1 werden durch eine Anpreßeinrichtung mit ihren Formtrennflächen 3 gegeneinander gepreßt. Diese Anpreßeinrichtung besteht beispielsweise aus mehreren Anpreßschrauben 7, die in den Formteilhälften senkrecht zu den Formtrennftächen 3 angebracht sind.

Ein Gehäuseboden 8 und zwei seitlich daran angeschraubte Seitenwände 9 und 10 bilden ein im Querschnitt U-förmiges Gehäusegrundteil, das mit einem abnehmbar befestigten Gehäusedeckel 11 verschlossen ist.

Die Formnester 4 sind jeweils mit einer an der oberen Formaußenfläche 12 bzw. Der unteren Formaußenfläche 13 mündenden Angußöffnung 14 verbunden. Diesen Angußöffnungen 14 liegen im Bereich der Formtrennfläche 3 und an den Stellen, an denen die kugelförmigen Formnester 4 jeweils den geringstmöglichen Abstand zu der jeweils benachbarten Formaußenfläche 12 bzw. 13 haben. Dadurch sind die Angußöffnungen 14 minimiert, so daß beim Herauslösen des fertigen Implantats praktische kein störender Anguß verbleibt.

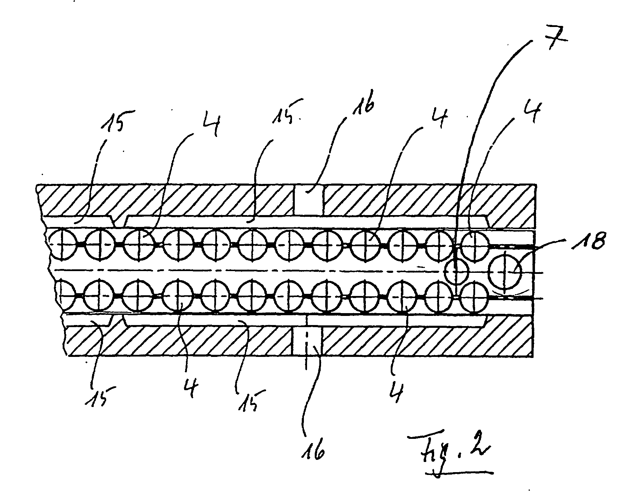

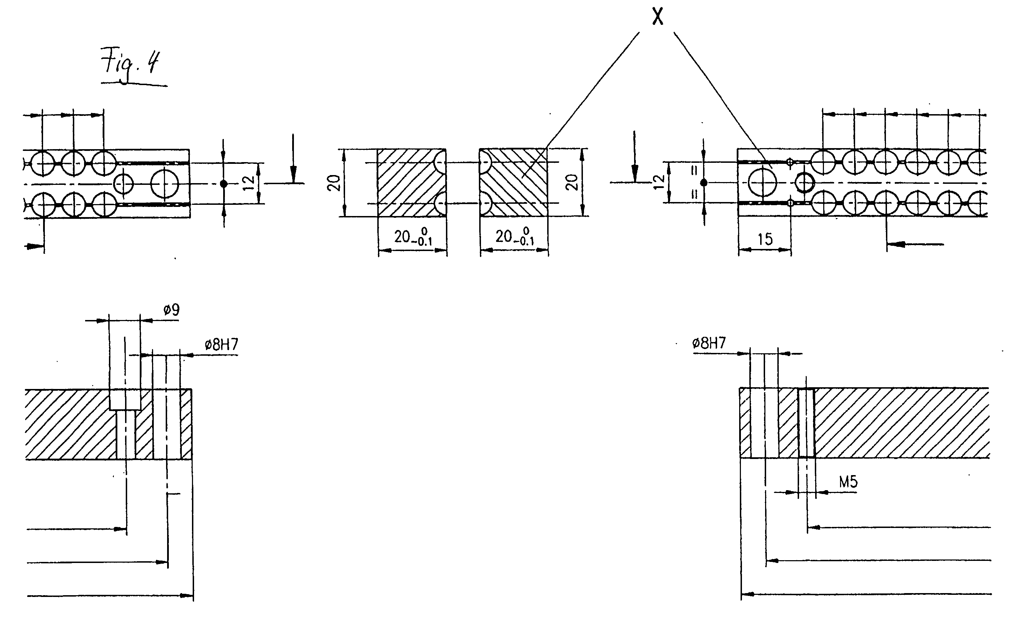

Wie man aus Fig. 2 erkennt, sind jeweils mehrere Formnester 4 einer Reihe an eine gemeinsame Verteilernut 15 angeschlossen, die in einer Innenwand des Gehäusedeckels 1 bzw. Des Gehäusebodens 8 ausgespart ist. In jede Verteilernut 15 mündet eine zur Gehäuseaußenseite führende Einspritzöffnung 16, die zur Durchführung des Spritzgießvorgangs mit einer Einspritzvorrichtung dicht verbunden werden kann, beispielsweise einer in Fig. 1 mit strichpunktierten Linien nur teilweise angedeuteten Befüllspritze 17. Für jede Reihe von Formnestern 4 sind mehrere, miteinander fluchtende Verteilernuten 15 vorgesehen, in die jeweils eine gesonderte Einspritzöffnung 16 mündet.

Nach dem Einlegen des chirurgischen Fadens 6 oder Drahtes werden die beiden Formhälften 1 mittels der Positionsreinrichtung 18 in die richtige Lage gebracht, durch die Anpreßvorrichtung 7 gegeneinander bis auf Dichtigkeit gedrückt, um die Form zu schließen, und in das Gehäuse 2 eingeführt und sodann werden alle oder die jeweils gewünschte Anzahl von Formnestern 4 mittels der nacheinander an die Einspritzöffnungen 6 angeschlossenen Befüllspritze 17 mit dem fließfähigen Gemisch aus Knochenzement und Wirkstoff gefüllt. Nach dem Aushärten des Knochenzements wird zunächst die Deckelverschraubung des Formgehäuses gelöst, auf die Formteilhälften ein Kraftimpuls (z.B. Hammerschlag) ausgeübt, um die Formhälften vom Anguß abzuscheren und es werden die beiden Formhälften 1 herausgenommen. Nach Lösen der Anpreßvorrichtung 7 werden die Formteilhälften getrennt und die geformten Implantatketten entnommen. Der in den Verteilernuten 15 und in den Einspritzöffnungen 16 verbleibende Anguß kann in einfacher Weise entfernt werden, so daß die Vorrichtung unmittelbar danach wieder zusammengebaut und für einen weiteren Formvorgang verwendet werden kann.

## Patentansprüche

1. Vorrichtung zur Herstellung von perlschnurförmigen pharmakahaltigen Implantaten, bestehend aus Ketten von jeweils auf einem chirurgischen Draht oder Faden (6) aufgereihten pharmakahaltigen Formkörperchen, **dadurch gekennzeichnet, daß**
(i) zwei längliche Formhälften (1) in einem Formgehäuse (2) aufgenommen, mittels einer Positionierungseinrichtung (18) gegeneinander ausgerichtet und durch eine Anpreßeinrichtung (7) mit ihren Formtrennflächen (3) gegeneinander bis auf Dichtigkeit preßbar sind, daß
(ii) in den einander zugekehrten Formtrennflächen (3) mindestens in einer Reihe angeordnete Formnester (4) zur Aufnahme jeweils eines Formkörperchens und eines diese verbindenden Drahtes oder Fadens (6) ausgespart sind, daß
(iii) die Formnester (4) jeweils mit einer an einer Formaußenfläche (12 bzw. 13) mündenden Angußöffnung (14) verbunden sind, und daß
(iv) jeweils mehrere Formnester (4) einer Reihe an eine gemeinsame, in einer Innenwand des Formgehäuses ausgesparte Verteilernut (15) angeschlossen sind, in die jeweils eine mit einer Einspritzvorrichtung (1) dicht verbindbare Einspritzöffnung (16) mündet.

2. Vorrichtung nach Anspruch 1, **dadurch gekennzeichnet, daß** die Angußöffnungen (14) der Formnester (4) jeweils im Bereich der Formtrennfläche (3) und an den Stellen des geringstmöglichen Abstandes zwischen dem Formnest (4) und der Formaußenfläche (12 bzw. 13) angeordnet sind.

3. Vorrichtung nach Anspruch 1, **dadurch gekennzeichnet, daß** in den beiden Formhälften (1) zwei parallele Reihen von Formnestern (4) ausgespart sind, deren Angußöffnungen (14) in gegenüberliegenden Formaußenflächen (12 bzw. 13) münden, und daß die Verteilernuten (15) und die Einspritzöffnungen (16) in einander gegenüberliegenden Wänden (8, 11) des Formgehäuses (2) angeordnet sind.

4. Vorrichtung nach Ansprüchen 1 oder 3, **dadurch gekennzeichnet, daß** für jede Reihe von Formnestern (4) mehrere, miteinander fluchtende Verteilernuten (15) vorgesehen sind, in die jeweils eine gesonderte Einspritzöffnung (16) mündet.

5. Vorrichtung nach Anspruch 1, **dadurch gekennzeichnet, daß** das Formgehäuse (2) aus einem im Querschnitt U-förmigen Gehäusegrundteil (8, 9, 10) und einem abnehmbar befestigten Gehäusedeckel (11) besteht.

6. Vorrichtung nach Anspruch 1, **dadurch gekennzeichnet, daß** die Anpreßeinrichtung (7) aus mehreren versenkten Verschraubungen senkrecht zur Formtrennfläche (3) der Formhälften (1) in den Formhälften selbst besteht.

7. Vorrichtung nach Anspruch 1, **dadurch gekennzeichnet, daß** die Positionierungseinrichtung (18) aus mindestens zwei bolzenähnlichen Stiften auf der eine Formhälfte (1) senkrecht zur Formtrennfläche (3) und korrespondierenden Vertiefungen auf der anderen Formhälfte (1) besteht.

## Claims

1. Apparatus for the production of drug-containing implants in the form of strings of beads, comprising chains of small drug-containing moulded bodies respectively arranged in series on a surgical wire or thread (6), **characterized in that**
(i) two elongate mould halves (1) are accommodated in a mould housing (2), are aligned with respect to one another by means of a positioning device (18) and are adapted such that they can be pressed until sealed against each other with their mould parting surfaces (3) by a pressure-exerting means (7), **in that**
(ii) there are made in the mutually facing mould parting surfaces (3) mould cavities (4), arranged at least in one row, for receiving in each case a small moulded body and a wire or thread (6) joining the said bodies, **in that**
(iii) the mould cavities (4) are respectively connected to a gate (14) opening out at a mould outer surface (12 or 13), and **in that**
(iv) in each case a plurality of mould cavities (4) of a row are connected to a common runner (15) which is made in an inner wall of the mould housing and into which there respectively opens out an injection opening (16) adapted such that it can be connected in a sealed manner to an injection device (1).

2. Apparatus according to Claim 1, **characterized in that** the gates (14) of the mould cavities (4) are arranged in each case in the region of the mould parting surface (3) and at the points of least possible distance between the mould cavity (4) and the mould outer surface (12 or 13).

3. Apparatus according to Claim 1, **characterized in that** in the two mould halves (1) there are made two parallel rows of mould cavities (4), the gates (14) of which open out in opposing mould outer surfaces (12 and 13, respectively), and **in that** the runners (15) and the injection openings (16) are arranged in mutually opposing walls (8, 11) of the mould housing (2).

4. Apparatus according to Claims 1 or 3, **characterized in that** for each row of mould cavities (4) there are provided a plurality of mutually aligned runners (15), into which there opens out in each case a separate injection opening (16).

5. Apparatus according to Claim 1, **characterized in that** the mould housing (2) comprises a cross-sectionally U-shaped basic housing part (8, 9, 10) and a removably fastened housing cover (11).

6. Apparatus according to Claim 1, **characterized in that** the pressure-exerting means (7) comprises a plurality of recessed screwed joints perpendicular to the mould parting surface (3) of the mould halves (1) in the mould halves themselves.

7. Apparatus according to Claim 1, **characterized in that** the positioning device (18) comprises at least two bolt-like pins on one mould half (1), perpendicular to the mould parting surface (3), and corresponding depressions on the other mould half (1).

## Revendications

1. Dispositif pour la fabrication d'implants en forme de collier de perles, contenant des produits pharmaceutiques, constitués de chaînes de corpuscules moulés contenant des produits pharmaceutiques, enfilés chacun sur un fil ou fil métallique chirurgical, **caractérisé en ce que**
(I) deux demi-moules allongés (1) sont placés dans un boîtier de moule (2), orientés l'un par rapport à l'autre au moyen d'un dispositif de positionnement (18) et pouvant être pressés l'un contre l'autre jusqu'à l'étanchéité par leurs faces de séparation de moule (3) au moyen d'un dispositif de pression (7), **en ce que**
(II) dans les faces de séparation de moule (3) adjacentes l'une à l'autre, sont évidées des cavités de moule (4) disposées au moins en une série pour la réception chacune d'un corpuscule moulé et d'un fil ou fil métallique (6) les reliant, **en ce que**
(III) les cavités de moule (4) sont raccordées chacune à un orifice d'entrée (14) débouchant sur une face externe (12 ou 13) du moule, **en ce que**
(IV) plusieurs cavités de moule (4) dune série sont raccordées chacune à une gorge de distribution (15) commune, creusée dans une paroi interne du boîtier de moule, dans laquelle débouche chaque fois un orifice d'injection (16) pouvant être raccordé de façon étanche à un dispositif d'injection (1).

2. Dispositif selon la revendication 1, **caractérisé en ce que** les orifices d'entrée (14) des cavités de moule (4) sont disposés chacun dans la zone de la face de séparation (3) et aux points de la plus faible distance possible entre la cavité de moule (4) et la face externe de moule (12 ou 13).

3. Dispositif selon la revendication 1, **caractérisé en ce que** dans les deux demi-moules (1) sont évidées deux séries parallèles de cavités de moule (4) dont les orifices externes (14) débouchent dans des faces externes de moule (12 ou 13) opposées, et **en ce que** les gorges de distribution (15) et les orifices d'injection (16) sont disposés dans des parois (8, 11) du boîtier de moule (2) se faisant face.

4. Dispositif selon la revendication 1 ou 3, **caractérisé en ce que** pour chaque série de cavités de moule (4) sont prévues plusieurs gorges de distribution (15) alignées les unes avec les autres, dans chacune desquelles débouche un orifice d'injection spécial.

5. Dispositif selon la revendication 1, **caractérisé en ce que** le boîtier de moule (2) est constitué d'un élément de base (8, 9, 10) de boîtier, en forme de U en section transversale, et d'un couvercle (11) de boîtier fixé de façon amovible.

6. Dispositif selon la revendication 1, **caractérisé en ce que** le dispositif de pression (7) est constitué de plusieurs raccords par vis affleurant perpendiculairement à la surface de séparation de moule (3) des demi-moules (1) dans les demi-moules eux-mêmes.

7. Dispositif selon la revendication 1, **caractérisé en ce que** le dispositif de positionnement (18) est constitué d'au moins deux broches en forme de boulons sur un demi-moule (1), perpendiculaires à la surface de séparation de moule (3) et d'évidements correspondants sur l'autre demi-moule (1).
